# FASCICULE DE BREVET EUROPEEN

(11) **EP 1 599 140 B1**
(45) Date de publication et mention de la délivrance du brevet: **06.04.2011**
(21) Numéro de dépôt: 04715361.4
(22) Date de dépôt: 27.02.2004
(51) Int. Cl.: A61B 17/02

(54) **DISTRACTEUR DU GENOU**
KNIEGELENKDISTRAKTOR
KNEE DISTRACTOR

(30) Priorité: 27.02.2003 FR 0302413
(43) Date de publication de la demande: 30.11.2005
(73) Titulaire: UNIVERSITE JOSEPH FOURIER, 38041 Grenoble Cédex 9 (FR); Perception Raisonnement Action En Medecine, 38700 La Tronche (FR)
(72) Inventeur: CINQUIN, Philippe, F-38330 SAINT NAZAIRE LES EYMES (FR); MARMIGNON, Christophe, 66310 Estagel (FR); LAVALLEE, Stéphane, F-38410 Saint Martin d'Uriage (FR)
(74) Mandataire: de Beaumont, Michel
(86) Numéro de dépôt international: PCT/FR2004/050089
(87) Numéro de publication internationale: WO 2004/078047

(56) Documents cités:
- WO-A-95/20362
- WO-A-99/59669
- WO-A-02/102254
- WO-A-03/003951

## Description

La présente invention concerne un distracteur de genou, utilisé lors d'une opération d'implantation d'une prothèse du genou.

Lors d'une opération d'implantation, le chirurgien doit choisir la prothèse la plus adaptée de façon que le patient récupère la plus grande mobilité possible. Le choix de la prothèse est en partie déterminé par l'état du système capsuloligamentaire du genou. Pour obtenir une répartition optimale des efforts sur la prothèse, celle-ci doit être implantée parfaitement dans l'alignement de la jambe en extension et l'espace articulaire, c'est-à-dire la région séparant le fémur du tibia après réalisation des coupes osseuses, doit avoir une hauteur, correspondant à la plus petite distance séparant le fémur du tibia, identique en extension et pour une flexion à 90° de la jambe. L'égalité de hauteurs de l'espace articulaire assure une répartition acceptable des efforts sur les ligaments au cours d'un mouvement de flexion de la jambe.

Par ailleurs, étant donné la détérioration du genou, certains ligaments peuvent s'être rétractés par rapport à un état normal. Le chirurgien doit donc estimer au préalable le comportement du système ligamentaire avant l'implantation de la prothèse. En particulier, le chirurgien peut être amené à "relâcher" certains ligaments avant l'implantation, c'est-à-dire à inciser des ligaments pour leur permettre d'atteindre une plus grande longueur.

Pour évaluer la hauteur de l'espace articulaire et l'état du système ligamentaire, le chirurgien utilise un instrument médical appelé distracteur qui permet d'écarter les parties osseuses de l'articulation du genou afin de mesurer la hauteur de l'espace articulaire tout en mettant en tension le système ligamentaire.

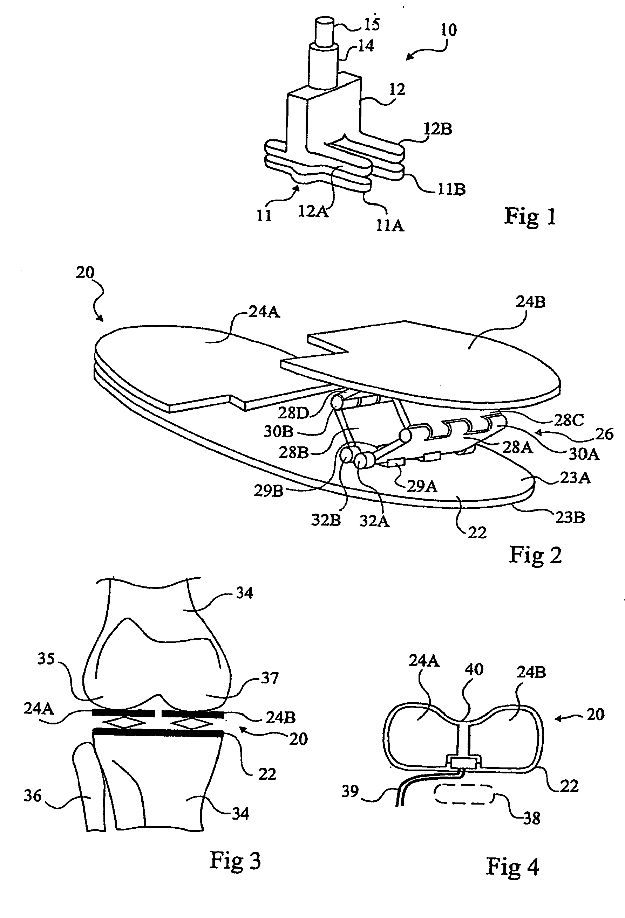

La figure 1 représente, de façon schématique, un exemple de distracteur classique 10. Le distracteur 10 comprend une embase 11 de laquelle s'étendent deux bras inférieurs 11A, 11B destinés à reposer sur l'extrémité découpée du tibia. Le distracteur 10 comprend un corps 12 adapté à coulisser par rapport à l'embase 11 selon une direction sensiblement perpendiculaire au plan médian des bras inférieurs 11A, 11B. Le corps 12 comprend deux bras supérieurs 12A, 12B sensiblement parallèles aux bras inférieurs 11A, 11B. Chaque bras supérieur 12A, 12B est destiné à venir en contact avec un condyle du fémur. Un moyen de réglage 14, comportant une vis de réglage 15, est adapté à faire coulisser le corps 12 par rapport à l'embase 11 de façon à régler la position des bras supérieurs 12A, 12B par rapport aux bras inférieurs 11A, 11B.

Pour mettre en place le distracteur 10, le genou est maintenu en extension et le chirurgien réalise une incision. La rotule est mise en éversion, c'est-à-dire qu'elle est déplacée latéralement par rapport à sa position normale et retournée, afin de libérer une place suffisante pour réaliser la coupe de l'extrémité supérieure du tibia et mettre en place le distracteur 10. Les bras inférieurs 11A, 11B et supérieurs 12A, 12B sont introduits dans l'espace articulaire ainsi libéré par l'avant de l'articulation, les bras inférieurs 11A, 11B étant placés en contact avec l'extrémité découpée du tibia. Le corps 12 du distracteur 10 est déplacé par rapport à l'embase 11 jusqu'à ce que les bras supérieurs 12A, 12B viennent en contact avec les condyles du fémur. On mesure alors la hauteur de l'espace articulaire.

Pendant l'utilisation du distracteur 10, une partie du corps 12 et de l'embase 11 est en saillie à l'extérieur de l'espace articulaire par l'avant de l'articulation du genou. Un inconvénient est qu'il est par conséquent nécessaire de maintenir la rotule en éversion pendant toute l'utilisation du distracteur.

On peut donc obtenir une mesure de l'espace articulaire et une appréciation indirecte et qualitative des efforts exercés sur le système ligamentaire seulement lorsque le genou est en extension. Pour obtenir une estimation de l'espace articulaire pour une autre position de flexion de la jambe, généralement 90°, le chirurgien doit retirer le distracteur 10 de l'espace articulaire, remettre la rotule en position normale, et enfin fléchir la jambe à la position souhaitée. Le chirurgien relâche les ligaments si cela est nécessaire jusqu'à insérer à nouveau dans l'espace articulaire le distracteur dont l'épaisseur correspond à l'épaisseur de l'espace articulaire mesurée par le distracteur pour la jambe en extension.

Un tel distracteur ne permet donc pas de suivre l'évolution de l'espace articulaire et des efforts exercés sur les différents éléments de l'articulation en fonction de la flexion de la jambe. Le choix de la prothèse est donc habituellement réalisé de façon empirique à partir d'une seule mesure directe de la géométrie de l'espace articulaire. De plus, dans le cas où un relâchement des ligaments est nécessaire, celui-ci est réalisé en prenant en compte uniquement les efforts exercés sur le système ligamentaire pour une flexion déterminée, généralement à 90° ce qui peut s'avérer être insatisfaisant.

La demande de brevet WO-A-2520362 divulgue des distracteurs du genou comprenant des ballons gonflables, avec une surface inférieure et une surface supérieure, et des conduites souples d'apport du fluide qui écarte lesdites surfaces l'une de l'autre à différentes positions de flexion.

La présente invention vise un distracteur permettant l'écartement de parties osseuses d'une articulation du genou préalablement à une implantation de prothèse pour la réalisation de mesures de distances et d'efforts caractéristiques de l'articulation à différentes positions de flexion de la jambe.

Pour atteindre cet objet, la présente invention prévoit un instrument médical comprenant un plateau inférieur ; au moins un plateau supérieur en vis-à-vis du plateau inférieur ; entre le plateau supérieur et le plateau inférieur, un moyen d'espacement pour maintenir en permanence le plateau supérieur sensiblement parallèle au plateau inférieur et pour écarter le plateau supérieur du plateau inférieur, ledit moyen étant contenu en totalité entre le plateau supérieur et le plateau inférieur, l'espacement minimal entre les faces externes du plateau supérieur et du plateau inférieur étant inférieur à 10 millimètres ; et des moyens de commande souples reliés au moyen d'espacement.

Selon un exemple de réalisation de l'invention, l'instrument médical comporte deux plateaux supérieurs disposés côte à côte et en vis-à-vis du plateau inférieur, chaque plateau supérieur étant associé à un moyen d'espacement.

Selon un exemple de réalisation de l'invention, l'instrument médical comporte un moyen pour mesurer la distance entre le plateau supérieur et le plateau inférieur.

Selon un exemple de réalisation de l'invention, l'instrument médical comporte un moyen pour mesurer les efforts exercés sur le plateau supérieur.

Selon un exemple de réalisation de l'invention, le moyen d'espacement comprend un système de liaison constitué de quatre plaques, deux plaques inférieures reliées au plateau inférieur par des liaisons à pivot adjacentes, et deux plaques supérieures reliées au plateau supérieur par des liaisons à pivot adjacentes, chaque plaque inférieure étant reliée à une plaque supérieure par une liaison à pivot, le système de liaison comprenant en outre un moyen pour maintenir les plaques inférieures sensiblement symétriques par rapport au plan médian perpendiculaire au plateau inférieur.

Selon un exemple de réalisation de l'invention, le moyen d'espacement comprend un moyen d'actionnement par câble et/ou un actionneur à fluide relié au plateau inférieur et au plateau supérieur.

Selon un exemple de réalisation de l'invention, la face externe du plateau supérieur est plane.

Selon un exemple de réalisation de l'invention, l'instrument médical est destiné à écarter des parties osseuses d'une articulation du genou, la rotule étant en position normale, le plateau inférieur étant destiné à être maintenu à une extrémité découpée du tibia, chaque plateau supérieur étant destiné à être en contact avec un condyle du fémur.

Selon un exemple de réalisation de l'invention, chaque moyen d'espacement est destiné à être commandé pour différentes positions de flexion de la jambe.

Selon un exemple de réalisation de l'invention, le plateau inférieur et les plateaux supérieurs comportent un enfoncement du côté opposé au côté destiné à être le plus proche de la rotule.

Cet objet, ces caractéristiques et avantages, ainsi que d'autres de la présente invention seront exposés en détail dans la description suivante d'exemples de réalisation particuliers faite à titre non-limitatif en relation avec les figures jointes parmi lesquelles :
la figure 1, précédemment décrite, représente de façon schématique une vue en perspective d'un distracteur classique ;
la figure 2 représente de façon schématique une vue partielle en perspective d'un exemple de réalisation d'un distracteur selon l'invention ;
la figure 3 représente, de façon schématique, une vue de face d'un distracteur mis en place au niveau d'une articulation de genou ; et
la figure 4 représente, de façon schématique, une vue de dessus du distracteur de la figure 3.

Comme cela est représenté en figure 2, le distracteur 20 selon l'invention comprend un plateau inférieur 22 ayant des première et seconde faces 23A, 23B sensiblement planes et parallèles. La seconde face 23B du plateau inférieur 22 est destinée à être maintenue à l'extrémité découpée du tibia par des moyens d'ancrage non représentés. Le distracteur 20 comprend deux plateaux supérieurs 24A, 24B, sensiblement plans. Chaque plateau supérieur 24A, 24B est relié à la première face 23A du plateau inférieur 22 par un système d'espacement 26 (un seul système d'espacement étant visible en figure 2). Le système d'espacement 26 permet le déplacement du plateau supérieur 24A, 24B associé par rapport au plateau inférieur 22 tout en maintenant en permanence le plan moyen du plateau supérieur 24A, 24B sensiblement parallèle à la première face 23A du plateau inférieur 22.

Selon le présent exemple de réalisation, le système d'espacement 26 reliant une plaque supérieure 24A, 24B au plateau inférieur 22 comprend quatre plaques 28A à 28D disposées en forme de parallélépipède, deux plaques inférieures 28A, 28B reliées chacune au plateau inférieur 22 par une liaison à pivot inférieure 29A, 29B, et deux plaques supérieures reliées chacune au plateau supérieur 24A, 24B par une liaison à pivot (non visible en figure 2). Chaque plaque inférieure 28A, 28B est reliée à une plaque supérieure 28C, 28D par une liaison à pivot supérieure 30A, 30B. Les liaisons à pivot inférieures 29A, 29B sont adjacentes et sont associées à des roues dentées 32A, 32B pour assurer un débattement symétrique des deux plaques inférieures 28A, 28B par rapport à un plan médian perpendiculaire au plateau inférieur 22. De façon analogue, les liaison à pivot supérieures sont adjacentes et sont associées à des roues dentées (non visibles en figure 2) pour assurer un débattement symétrique des deux plaques supérieures 28C, 28D par rapport à un plan médian perpendiculaire au plateau supérieur 24A, 24B. La structure du système d'espacement 26 est telle qu'il est contenu en totalité entre le plateau supérieur 24A, 24B et le plateau inférieur 22.

A titre d'exemple, le plateau supérieur 24A, représenté en figure 2 sur la gauche, est dans la position la plus proche du plateau inférieur 22. Le plateau supérieur 24B représenté en figure 2 sur la droite, est dans une position à une distance du plateau inférieur 22 intermédiaire entre la distance minimale et la distance maximale pouvant être atteinte. A titre d'exemple, lorsque les deux plateaux supérieurs 24A, 24B sont à une distance maximale du plateau inférieur 22, l'épaisseur totale du distracteur 20 selon l'invention est d'environ 20 millimètres, et lorsque les deux plateaux supérieurs 24A, 24B sont à une distance minimale du plateau inférieur 22, l'épaisseur totale du distracteur 20 selon l'invention est sensiblement inférieure à 10 millimètres, et de façon avantageuse inférieure à 5 millimètres.

Le système d'espacement 26 comprend également pour chaque plateau supérieur 24A, 24B un moyen d'actionnement (non représenté) adapté à écarter le plateau supérieur 24A, 24B du plateau inférieur 22. Les moyens d'actionnement associés aux plateaux supérieurs 24A, 24B peuvent être actionnés indépendamment l'un de l'autre de façon à déplacer les plateaux supérieurs 24A, 24B par rapport au plateau inférieur 22 indépendamment l'un de l'autre. Selon un exemple de réalisation, le moyen d'actionnement est un système à câbles. Selon un autre exemple de réalisation, le moyen d'actionnement comprend un actionneur à fluide, par exemple un piston pneumatique, placé entre le plateau supérieur associé 24A, 24B et le plateau inférieur 22. L'injection d'un fluide, par exemple de l'air comprimé, au niveau de l'actionneur assure l'éloignement du plateau supérieur 24A, 24B par rapport au plateau inférieur 22.

Un capteur de distance (non représenté), agencé au niveau de chaque plateau supérieur 24A, 24B, est adapté à mesurer la distance séparant le plateau supérieur 24A, 24B du plateau inférieur 22. Il s'agit par exemple d'un capteur à effet Hall associé à un aimant permanent disposé au niveau du plateau inférieur 22. Un capteur d'effort (non représenté), également agencé au niveau de chaque plateau supérieur 24A, 24B, est adapté à mesurer les efforts exercés sur le plateau supérieur 24A, 24B par le système d'espacement 26. Les capteurs de distance et d'effort sont, par exemple, directement intégrés aux plateaux ou sont rapportés sur ceux-ci.

La figure 3 représente de façon schématique le distracteur 20 selon l'invention mis en place dans l'espace articulaire du genou entre le tibia 34 et le fémur 35, une fois la coupe tibiale réalisée. A titre d'illustration, le péroné 36 est également représenté. Comme cela est représenté, le plateau inférieur 22 du distracteur 20 est fixé à l'extrémité découpée du tibia 34 et les plateaux supérieurs 24A, 24B du plateau inférieur 22 sont en contact avec les condyles 37A, 37B du fémur 35. Etant donné les formes différentes des condyles 37A, 37B, les plateaux supérieurs 24A, 24B ne sont généralement pas écartés d'une même distance du plateau inférieur 22.

La figure 4 représente une vue de dessus du distracteur 20 de la figure 3. On a représenté en traits pointillés l'emplacement de la rotule 38. Des câbles et/ou des conduites 39 relient le distracteur 20 à un module de traitement et de commande. Les câbles ou conduites 39 transmettent au module de traitement et de commande les données fournies par les capteurs d'effort ou de distance et fournissent au distracteur 20 la commande des systèmes d'espacement 26. A titre d'exemple, lorsque le système d'espacement 26 comprend un actionneur à fluide, une conduite 39 est une conduite d'apport en fluide. Le distracteur 20 selon l'invention est tel qu'aucun élément ne se projette en saillie depuis la région située entre les plateaux supérieurs 24A, 24B et le plateau inférieur 22 quelles que soient les positions des plateaux supérieurs 24A, 24B, à l'exception des câbles et des conduites 39. Le distracteur 20 selon l'invention occupe donc une place minimale. De plus, les câbles et les conduites 39 sont suffisamment souples pour être plaqués le long du distracteur 20. Le distracteur 20 selon l'invention peut donc être utilisé tout en maintenant la rotule 38 dans sa position normale.

En outre, le plateau inférieur 22 et les plateaux supérieurs 24A, 24B comprennent un enfoncement 40 du côté opposé à la rotule 38 de façon à ne pas gêner le passage des veines, d'artères, de nerfs, de ligaments, etc., dans cette partie de l'articulation du genou.

Le distracteur 20 selon l'invention est mis en place au niveau de l'articulation du genou de la façon suivante.

Une incision permettant l'accès à l'articulation est réalisée. La rotule 38 est alors mise en éversion et la coupe tibiale est effectuée. Ceci libère l'espace articulaire entre l'extrémité découpée du tibia 34 et les condyles 37A, 37B du fémur 35 qui a une hauteur généralement comprise entre 5 et 10 millimètres. Lors de la coupe tibiale, les éléments qui seront remplacés par la prothèse sont également supprimés, à savoir le ménisque, et selon leur état, certains ligaments, par exemple les ligaments croisés antérieurs et postérieurs. Le distracteur 20 selon l'invention est alors inséré dans l'espace ainsi libéré. L'insertion du distracteur 20 selon l'invention est facilitée par le fait que chaque plateau supérieur 24A, 24B est dans la position la plus proche du plateau inférieur 22. Le distracteur 20 a donc une épaisseur totale faible. Le plateau inférieur 22 du distracteur 20 est fixé à l'extrémité découpée du tibia 34. La rotule 38 est ensuite remise en position normale. On écarte alors, par l'intermédiaire des systèmes d'espacement 26, les plateaux supérieurs 24A, 24B du plateau inférieur 22 jusqu'à ce qu'ils soient en contact avec les condyles 37A, 37B du fémur 35. L'ensemble des éléments de l'articulation est alors mis plus ou moins en tension.

Deux modes de mise en tension sont envisageables. Premièrement, il est possible d'imposer les valeurs des efforts appliqués au niveau des plateaux supérieurs 24A, 24B, c'est-à-dire globalement appliqués au système ligamentaire, au moyen des capteurs d'effort. On mesure alors la distance séparant chaque plateau supérieur 24A, 24B du plateau inférieur 22 par l'intermédiaire du capteur de distance. Deuxièmement, il est possible d'imposer les distances séparant les plateaux supérieurs 24A, 24B du plateau inférieur 22. On mesure alors les efforts qui sont alors supportés par les plateaux supérieurs 24A, 24B et qui sont exercés sur les efforts globalement exercés sur le système ligamentaire. Les plateaux supérieurs 24A, 24B peuvent être actionnés indépendamment l'un de l'autre. On peut donc imposer des distances séparant les plateaux supérieurs 24A, 24B du plateau inférieur 22 qui sont différentes pour les deux plateaux supérieurs. De façon analogue, on peut imposer des efforts appliqués au niveau des plateaux supérieurs 24A, 24B qui sont différents pour les deux plateaux supérieurs.

Les mesures d'efforts et de distances sont effectuées pour différentes positions de flexion de la jambe. Les deux modes de mise en tension de l'articulation permettent notamment de contrôler une mesure en imposant alternativement la distance ou l'effort. Les différentes mesures obtenues pour différentes positions de flexion de la jambe permettent au chirurgien d'apprécier l'évolution de l'espace articulaire et des efforts exercés sur le système ligamentaire pour l'ensemble du mouvement de flexion de la jambe et aident donc le chirurgien dans son choix de la prothèse la plus adaptée. De plus, le distracteur selon la présente invention permet au chirurgien de pratiquer, à une ou à différentes positions de flexion de la jambe, un relâchement ligamentaire tout en contrôlant l'effet du relâchement à l'aide des informations fournies par le distracteur.

Le distracteur selon l'invention comporte une forme compacte puisque les systèmes d'espacement sont contenus en totalité entre les plateaux supérieurs et le plateau inférieur. En outre, les câbles et les conduites reliés au distracteur sont souples pour être facilement déplacés et ne pas gêner les mouvements de parties osseuses de l'articulation du genou. Un avantage de la présente invention est de pouvoir utiliser le distracteur en maintenant la rotule de l'articulation du genou en position normale. Le distracteur peut donc être utilisé pour différentes positions de flexion de la jambe.

En outre, le distracteur selon la présente invention permet de tenir compte de l'espace femoro-patellaire lors de la détermination d'un emplacement idéal pour l'implantation de la prothèse tibiale puisque les mesures de mise en tension sont réalisées lorsque la rotule est en place. Il peut donc être réalisé parallèlement des mesures pour déterminer le déplacement de la rotule et mesurer sa forme de façon à implanter une prothèse patellaire ayant la même trajectoire que la rotule.

Selon une variante de l'invention, le distracteur ne comprend pas de capteurs de distance. La mesure des distances entre les plateaux supérieurs et le plateau inférieur, si elle est désirée, peut se déduire des positions du tibia et du fémur. En effet, lors de certaines opérations chirurgicales, les positions du fémur et du tibia sont déterminées par ailleurs, par exemple par l'intermédiaire de corps rigides fixés au fémur et au tibia, notamment pour la détermination de plans de coupe.

La présente invention a été décrite dans le cadre d'un distracteur comportant deux plateaux supérieurs disposés côte à côte et en vis-à-vis du plateau inférieur et destinés à venir chacun en contact avec un condyle du fémur. Il est clair que le distracteur pourrait ne comprendre qu'un unique plateau supérieur disposé en vis-à-vis du plateau inférieur. Un tel distracteur est adapté pour la réalisation d'une prothèse du type unicompartimentale. Il s'agit par exemple d'une prothèse du genou dans laquelle seule une portion de l'extrémité du tibia est découpée. Le plateau inférieur est alors destiné à être fixé au niveau de la portion découpée du tibia et l'unique plateau supérieur est destiné à venir en contact avec le condyle du fémur en vis-à-vis de la portion découpée du tibia.

Bien entendu, la présente invention est susceptible de diverses variantes et modifications qui apparaîtront à l'homme de l'art. En particulier, les plateaux supérieurs peuvent être maintenus sensiblement parallèles au plateau inférieur par un mécanisme à câbles.

## Revendications

1. Instrument médical (20), destiné à une opération du genou, comprenant :
- un plateau inférieur (22) ;
- au moins un plateau supérieur (24A, 24B) en vis-à-vis du plateau inférieur ;
- entre le plateau supérieur et le plateau inférieur, un moyen d'espacement (26) pour maintenir en permanence le plateau supérieur sensiblement parallèle au plateau inférieur et pour écarter le plateau supérieur du plateau inférieur, ledit moyen étant contenu en totalité entre le plateau supérieur et le plateau inférieur à l'exception d'éventuels câbles et/ou conduites, l'espacement minimal entre les faces externes du plateau supérieur et du plateau inférieur étant inférieur à 10 millimètres ; et
- des moyens de commande souples reliés au moyen d'espacement.

2. Instrument médical selon la revendication 1, comportant deux plateaux supérieurs (24A, 24B) disposés côte à côte et en vis-à-vis du plateau inférieur (22), chaque plateau supérieur étant associé à un moyen d'espacement (26).

3. Instrument médical selon la revendication 1, comportant un moyen pour mesurer la distance entre le plateau supérieur (24A, 24B) et le plateau inférieur (22).

4. Instrument médical selon la revendication 1, comportant un moyen pour mesurer les efforts exercés sur le plateau supérieur (24A, 24B).

5. Instrument médical selon la revendication 1, dans lequel le moyen d'espacement (26) comprend un système de liaison constitué de quatre plaques (28A, 28B, 28C, 28D), deux plaques inférieures reliées au plateau inférieur (22) par des liaisons à pivot (29A, 29B) adjacentes, et deux plaques supérieures reliées au plateau supérieur par des liaisons à pivot adjacentes, chaque plaque inférieure étant reliée à une plaque supérieure par une liaison à pivot (30A, 30B), le système de liaison comprenant en outre un moyen pour maintenir les plaques inférieures sensiblement symétriques par rapport au plan médian perpendiculaire au plateau inférieur.

6. Instrument médical selon la revendication 1, dans lequel le moyen d'espacement (26) comprend un moyen d'actionnement par câble et/ou un actionneur à fluide relié au plateau inférieur (22) et au plateau supérieur.

7. Instrument médical selon la revendication 1, dans lequel la face externe du plateau supérieur (24A, 24B) est plane.

8. Instrument médical selon la revendication 2, dans lequel le plateau inférieur (22) et les plateaux supérieurs (24A, 24B) comportent un enfoncement (40) du côté opposé au côté destiné à être le plus proche de la rotule (38).

## Claims

1. A medical instrument (20) for a knee operation comprising:
- a lower element (22);
- at least one upper element (24A, 24B) facing the lower element;
- between the upper element and the lower element, spacing means (26) for permanently maintaining the upper element substantially parallel to the lower element and for spacing apart the upper element from the lower element, said means being, at the exception of possible cables or ducts, totally contained between the upper element and the lower element, the minimum interval between the external surfaces of the upper element and of the lower element being smaller than 10 millimeters; and
- flexible control means connected to the spacing means.

2. The medical instrument of claim 1, comprising two upper elements (24A, 24B) arranged side-by-side and facing the lower element (22), each upper element being associated with spacing means (26).

3. The medical instrument of claim 1, comprising means for measuring the distance between the upper element (24A, 24B) and the lower element (22).

4. The medical instrument of claim 1, comprising means for measuring the forces exerted on the upper element (24A, 24B).

5. The medical instrument of claim 1, in which the spacing means (26) comprise a connection system formed of four links (28A, 28B, 28C, 28D), two lower links connected to the lower element (22) by adjacent pivotal connections (29A, 29B), and two upper links connected to the upper element by adjacent pivotal connections, each lower link being connected to an upper link by a pivotal connection (30A, 30B), the connection system further comprising means for maintaining the lower links substantially symmetrical with respect to the median plane perpendicular to the lower element.

6. The medical instrument of claim 1, in which the spacing means (26) comprise a cable actuation means and/or a fluid actuator connected to the lower element (22) and to the upper element.

7. The medical instrument of claim 1, in which the external surface of the upper element (24A, 24B) is planar.

8. The medical instrument of claim 2, in which the lower element (22) and the upper elements (24A, 24B) comprise a recess (40) on the side opposite to the side intended to be closer to the patella (38).

## Patentansprüche

1. Ein medizinisches Instrument (20) für eine Knieoperation, das Folgendes aufweist:
ein unteres Element (22);
wenigstens ein oberes Element (24A, 24B), das zu dem unteren Element weist;
Abstandsmittel (26) zwischen dem oberen Element und dem unteren Element zum permanenten Beibehalten des oberen Elements im Wesentlichen parallel zu dem unteren Element und zum Beabstanden des oberen Elements von dem unteren Element, wobei die Mittel mit Ausnahme von möglichen Kabeln oder Kanälen vollständig zwischen dem oberen Element und dem unteren Element enthalten sind, wobei das minimale Intervall zwischen den Außenoberflächen des oberen Elements und des unteren Elements kleiner ist als 10 Millimeter; und
flexible Steuermittel, die mit den Abstandsmitteln verbunden sind.

2. Medizinisches Instrument nach Anspruch 1, das zwei obere Elemente (24A, 24B) aufweist, die Seite an Seite zueinander angeordnet sind und zu dem unteren Element (22) weisen, wobei jedes obere Element mit Abstandsmitteln (26) assoziiert ist.

3. Medizinisches Instrument nach Anspruch 1, das Mittel aufweist zum Messen des Abstands zwischen dem oberen Element (24A, 24B) und dem unteren Element (22).

4. Medizinisches Element nach Anspruch 1, das Mittel aufweist zum Messen der Kräfte, die auf das obere Element (24A, 24B) ausgeübt werden.

5. Medizinisches Instrument nach Anspruch 1, wobei die Abstandsmittel (26) ein Verbindungssystem aufweisen, das aus vier Verbindungen bzw. Gelenken (28A, 28B, 28C, 28D) aufgebaut ist, wobei zwei untere Verbindungen mit dem unteren Element (22) durch benachbarte Schwenkverbindungen (29A, 29B) verbunden sind und zwei obere Verbindungen mit dem oberen Element durch benachbarte Schwenkverbindungen verbunden sind, wobei jede untere Verbindung mit einer oberen Verbindung über eine Schwenkverbindung (30A, 30B) verbunden ist, wobei das Verbindungssystem ferner Mittel aufweist zum Halten der unteren Verbindungen im Wesentlichen symmetrisch bezüglich der Mittelebene senkrecht zu dem unteren Element.

6. Medizinisches Instrument nach Anspruch 1, wobei die Abstandsmittel (26) Kabelbetätigungsmittel und/oder einen Fluidbetätiger aufweisen, die bzw. der mit dem unteren Element (22) und dem oberen Element verbunden sind bzw. ist.

7. Medizinisches Instrument nach Anspruch 1, wobei die Außenoberfläche des oberen Elements (24A, 24B) planar ist.

8. Medizinisches Instrument nach Anspruch 2, wobei das untere Element (22) und die oberen Elemente (24A, 24B) eine Ausnehmung (40) in der Seite aufweisen, die der Seite gegenüberliegt, die am nächsten an der Patella (38) liegen soll.
